# EUROPEAN PATENT APPLICATION

(11) **EP 4 624 447 A1**
(43) Date of publication of application: **01.10.2025**
(21) Application number: 25164483.7
(22) Date of filing: 18.03.2025
(51) Int. Cl.: C07C 37/18, C07C 41/18, C07C 41/20, C07C 41/30, C07C 39/40, C07C 39/23, C07C 43/20, C07C 43/225, C07C 43/215

(54) **METHODS OF MAKING AND USING 1-METHYLEFFUSOL**

(30) Priority: 26.03.2024 US 202463569999 P
(71) Applicant: Laboratory for Synthetic Chemistry and Chemical Biology Limited, Hong Kong Shatin (HK)
(72) Inventor: MING, Chi, Units 1503-1511,15/F.,Building 17W, Hong Kong (HK); YIP, Kai, Units 1503-1511,15/F.,Building 17W, Hong Kong (HK)
(74) Representative: HGF

(57) **Abstract**

Methods of making 1-methyleffusol are described herein. The 1-methyleffusol compound, and pharmaceutical compositions thereof, can be used to stimulate bone forming activities in a subject in need thereof.

## Description

### CROSS-REFERENCED TO RELATED APPLICATIONS

This application claims the benefit of and priority to U.S. Provisional Application No. 63/569,999, filed March 26, 2024, which is hereby incorporated herein by reference in its entirety.

### FIELD OF THE INVENTION

This invention is generally in the field of methods of making and using a chemical compound, such as, for stimulating bone formation.

### BACKGROUND OF THE INVENTION

The naturally occurring compound 1,8-dimethyl-4-vinyl-9,10-dihydrophenanthrene-2,7-diol (or 1-Methyleffusol) can be obtained from the natural source, *Juncus effusus. J. effusus* is a Chinese herbal medicine used for the treatments of fidgetiness, insomnia, oliguria, painful difficult urination and with ulceration in the mouth or on the tongue.

However, 1-Methyleffusol exists in *J*. *effusus* only in minute quantities, within 0.75 to 5 ppm or within 0.000075 to 0.0005% by isolation (DellaGreca, M.; Fiorentino, A.; Isidori, M.; Lavorgna, M.; Monaco, P.; Previtera, L.; Zarrelli, A. Photochemistry 2002, 60, 633; Kúsz, N.; Stefkó, D.; Barta, A.; Kincses, A.; Szemerédi, N.; Spengler, G.; Hohmann, J.; Vasas, A. Molecules 2021, 26, 999; and Dellagreca, M.; Isidori, M.; Lavorgna, M.; Monaco, P.; Previtera, L.; Zarrelli, A. Journal of chemical Ecology 2004, 30, 867), which greatly hinders the availability of the compound for biological and pharmaceutical studies and uses.

Therefore, there remains a need to provide approaches to synthesize 1-Methyleffusol at sufficient yield and scale to address the issues with the compound when isolated from natural sources.

Therefore, it is the object of the present invention to provide methods to synthesize 1-Methyleffusol at larger scale.

It is a further object of the present invention to provide pharmaceutical compositions containing 1-Methyleffusol.

It is still a further object of the present invention to provide methods of using the pharmaceutical compositions for medical treatments.

### SUMMARY OF THE INVENTION

Methods of making 1,8-dimethyl-4-vinyl-9,10-dihydrophenanthrene-2,7-diol and pharmaceutical compositions thereof are described herein. Methods for using such pharmaceutical compositions, such as for stimulating bone formation in a subject in need thereof, are also described herein. In one non-limiting instance, a method of making 1,8-dimethyl-4-vinyl-9,10-dihydrophenanthrene-2,7-diol can include the steps of:
(i) refluxing 1-(bromomethyl)-3-methoxy-2-methylbenzene and triphenylphosphine in solution to afford a phosphonium bromide;
(ii) refluxing the phosphonium bromide and 3-methoxy-2-methylbenzaldehyde in an alkaline solution to afford (Z)- and (E)-1,2-bis(3-methoxy-2-methylphenyl)ethene stereoisomers;
(iii) hydrogenating the (Z)- and (E)-1,2-bis(3-methoxy-2-methylphenyl)ethene stereoisomers to afford 1,2-bis(3-methoxy- 2-methylphenyl)ethane;
(iv) brominating the 1,2-bis(3-methoxy- 2-methylphenyl)ethane to afford 5-bromo-1-methoxy-3-(3-methoxy-2- methylphenethyl)-2-methylbenzene;
(v) oxidatively coupling the 5-bromo-1-methoxy-3-(3-methoxy-2-methylphenethyl)-2-methylbenzene to afford 4-bromo-2,7-dimethoxy-1,8-dimethyl-9,10-dihydrophenanthrene;
(vi) demethylating the 4-bromo-2,7-dimethoxy-1,8-dimethyl-9,10-dihydrophenanthrene to afford 4-bromo-1,8-dimethyl-9,10-dihydrophenanthrene- 2,7-diol;
(vii) subjecting the 4-bromo-1,8-dimethyl-9,10-dihydrophenanthrene- 2,7-diol to a Suzuki-coupling reaction with a vinyl trifluoroborate salt and a palladium salt to afford 1,8-dimethyl-4-vinyl-9,10-dihydrophenanthrene-2,7-diol; and
(viii) purifying the 1,8-dimethyl-4-vinyl-9,10-dihydrophenanthrene-2,7-diol.
Scheme A below, shows a non-limiting example of the above method.

In some instances, the 1,8-dimethyl-4-vinyl-9,10-dihydrophenanthrene-2,7-diol (also denoted 1-Methyleffusol) compound formed according to these methods may be formed into a pharmaceutically acceptable salt.

In some instances, the 1-Methyleffusol compound synthesized forms part of a pharmaceutical composition. In one non-limiting instance, a pharmaceutical composition can include:
1,8-dimethyl-4-vinyl-9,10-dihydrophenanthrene-2,7-diol or a pharmaceutically acceptable salt thereof; and
one or more pharmaceutically acceptable carriers; and
optionally one or more pharmaceutically acceptable excipients.

The 1-Methyleffusol compound can act as an osteoblast agent. The compound is typically administered in a pharmaceutical composition to a subject in need thereof. In one non-limiting example, method of stimulating bone formation comprising administering a pharmaceutical composition including an effective amount of 1-Methyleffusol to a subject in need thereof. The method may also be used to promote bone formation in subjects which suffer from other diseases related with problems of the bone, such as weakening or weakened bones.

### BRIEF DESCRIPTION OF THE DRAWINGS

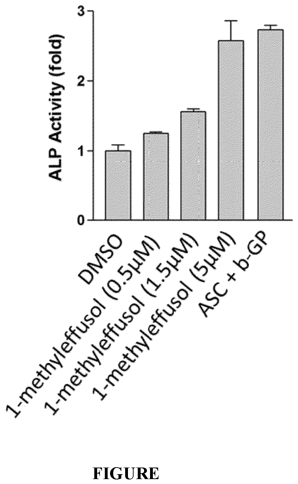

Non-limiting embodiments are described by way of example with reference to the accompanying Figure.

The **Figure** shows a graph of the stimulation of alkaline phosphatase (ALP) activity, as a marker of bone forming activity, in MC3T3-E1 osteoblasts by 1-Methyleffusol.

### DETAILED DESCRIPTION OF THE INVENTION

Methods of making 1,8-dimethyl-4-vinyl-9,10-dihydrophenanthrene-2,7-diol (also named as 1-methyleffusol) and pharmaceutical compositions thereof are described herein. In addition, methods for using the pharmaceutical compositions for stimulating bone formation in a subject in need thereof are also described herein.

### I. Definitions

It is to be understood that the disclosed compounds, compositions, and methods are not limited to specific synthetic methods, specific analytical techniques, or to particular reagents unless otherwise specified, and, as such, may vary. It is also to be understood that the terminology used herein is for the purpose of describing particular forms and embodiments only and is not intended to be limiting.

The term "subject," as used herein, refers to a mammal, such as a human.

The term "effective amount" or "therapeutically effective amount" refers to the amount of a compound or agent which is able to treat one or more symptoms of a disease or disorder, reverse the progression of one or more symptoms of a disease or disorder, halt the progression of one or more symptoms of a disease or disorder, or prevent the occurrence of one or more symptoms of a disease or disorder in a subject to whom the formulation is administered, for example, as compared to a matched subject not receiving the compound. The actual effective amounts of a compound can vary according to the specific compound or combination thereof being utilized, the particular composition formulated, the mode of administration, and the age, weight, condition of the individual, and severity of the symptoms or condition being treated.

The term "pharmaceutically acceptable" refers to compositions or other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" refers to pharmaceutically acceptable materials, compositions or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the subject to which it is administered.

"Pharmaceutically acceptable salt", as used herein, refers to derivatives of the 1-Methyleffusol compound defined herein, wherein the 1-Methyleffusol compound is modified by making acid or base salts thereof.

The terms "inhibit" or "reduce" in the context of inhibition, mean to reduce or decrease in activity and quantity. This can be a complete inhibition or reduction in activity or quantity, or a partial inhibition or reduction. Inhibition or reduction can be compared to a control or to a standard level. Inhibition can be measured as a % value, e.g., from 1% up to 100%, such as 5%, 10, 25, 50, 75, 80, 85, 90, 95, 99, or 100%. For example, pharmaceutical compositions including one or more types of active agents may inhibit or reduce one or more markers of a disease or disorder, such as a bacterial infection, in a subject by about 10%, 20%, 30%, 40%, 50%, 75%, 85%, 90%, 95%, or 99% from the activity and/or quantity of the same marker in subjects that did not receive or were not treated with the compositions.

The terms "treating" in the context of a disease or disorder means to ameliorate, reduce or otherwise stop a disease, disorder, or condition from occurring or progressing in an animal which may be predisposed to the disease, disorder and/or condition but has not yet been diagnosed as having it; inhibiting the disease, disorder or condition, e.g., impeding its progress; and relieving the disease, disorder, or condition, e.g., causing regression of the disease, disorder and/or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain. Desirable effects of treatment include decreasing the rate of disease progression, ameliorating, or palliating the disease state, and remission or improved prognosis. For example, a subject is successfully "treated" if one or more symptoms associated with a bacterial infection are mitigated or eliminated, including, but are not limited to, increasing the quality of life of those suffering from the disease, decreasing the dose of other medications required to treat the disease, delaying the progression of the disease, and/or prolonging survival of individuals.

The numerical ranges disclose individually each possible number that such a range could reasonably encompass, as well as any sub-ranges and combinations of sub-ranges encompassed therein. For example, in a given range concentration range of 10 µM to 500 µM, the range also discloses 20, 33, 350, and 499 µM, as well as any subrange between these numbers (for example, 50 µM to 250 µM), and any possible combination of ranges possible between these values.

Use of the term "about" is intended to describe values either above or below the stated value, which the term "about" modifies, to be within a range of approximately +/-10%. When the term "about" is used before a range of numbers (*i.e.,* about 1-5) or before a series of numbers (i.e., about 1, 2, 3, 4, etc.) it is intended to modify both ends of the range of numbers and/or each of the numbers recited in the entire series, unless specified otherwise.

### II. Methods of Making 1-Methyleffusol and Pharmaceutical Compositions Thereof

Described herein are methods of making 1,8-dimethyl-4-vinyl-9,10-dihydrophenanthrene-2,7-diol (also named as 1-methyleffusol) and pharmaceutical compositions thereof.

In one non-limiting instance, a method of making 1,8-dimethyl-4-vinyl-9,10-dihydrophenanthrene-2,7-diol includes the steps of:
(i) refluxing 1-(bromomethyl)-3-methoxy-2-methylbenzene and triphenylphosphine in solution to afford a phosphonium bromide;
(ii) refluxing the phosphonium bromide and 3-methoxy-2-methylbenzaldehyde in an alkaline solution to afford (Z)- and (E)-1,2-bis(3-methoxy-2-methylphenyl)ethene stereoisomers;
(iii) hydrogenating the (Z)- and (E)-1,2-bis(3-methoxy-2-methylphenyl)ethene stereoisomers to afford 1,2-bis(3-methoxy- 2-methylphenyl)ethane;
(iv) brominating the 1,2-bis(3-methoxy- 2-methylphenyl)ethane to afford 5-bromo-1-methoxy-3-(3-methoxy-2- methylphenethyl)-2-methylbenzene;
(v) oxidatively coupling the 5-bromo-1-methoxy-3-(3-methoxy-2-methylphenethyl)-2-methylbenzene to afford 4-bromo-2,7-dimethoxy-1,8-dimethyl-9,10-dihydrophenanthrene;
(vi) demethylating the 4-bromo-2,7-dimethoxy-1,8-dimethyl-9,10-dihydrophenanthrene to afford 4-bromo-1,8-dimethyl-9,10-dihydrophenanthrene- 2,7-diol;
(vii) subjecting the 4-bromo-1,8-dimethyl-9,10-dihydrophenanthrene- 2,7-diol to a Suzuki-coupling reaction with a vinyl trifluoroborate salt and a palladium salt to afford 1,8-dimethyl-4-vinyl-9,10-dihydrophenanthrene-2,7-diol; and
(viii) purifying the 1,8-dimethyl-4-vinyl-9,10-dihydrophenanthrene-2,7-diol.
Scheme A below, shows a non-limiting example of the above method.

Steps (i) and (ii) above represent a Wittig reaction where a phosphonium ylide is prepared from a phosphine and an alkyl halide and then reacted with a carbonyl compound (such as an aldehyde) in the presence of a strong base (such as alkoxides, LDA, or NaH) in a suitable solvent. The refluxing of steps (i) and (ii) occurs in a suitable solvent(s). Suitable solvents can be selected from, but are not limited to, water, toluene, dimethylformamide, dimethyl sulfoxide, acetonitrile, ethyl acetate, ether, hexane, tetrahydrofuran, and combinations thereof. In some instances, the suitable solvent in step (i) is toluene. In some instances, the suitable solvent in step (ii) includes water. In some instances, the suitable solvent in step (ii) is an aqueous alkaline solution which includes a salt, such as lithium chloride, and a base, such as lithium hydroxide.

In some instances, the hydrogenating of step (iii) includes reacting the (Z)- and (E)-1,2-bis(3-methoxy-2-methylphenyl)ethene stereoisomers with palladium on charcoal hydrogenation catalyst in one or more suitable solvents (such as, but not limited to, ethyl acetate, tetrahydrofuran, diethyl ether, ethanol, methanol, acetone, dichloromethane, isopropanol, and/or acetonitrile) under a hydrogen gas atmosphere. In some instances, the hydrogenation catalyst is a metal catalyst platinum (Pt) or nickel (Ni) on charcoal. Other hydrogenation conditions may be used, such as, but not limited to, the use of Wilkinson's catalyst.

In some instances, the brominating of step (iv) occurs by C-H borylation using an iridium(I) catalyst and subsequent bromination with a bromide salt, such as CuBr₂. C-H borylation conditions include the use of a transition metal catalyst (such as palladium, rhodium, or iridium based catalyst), a boron reagent (such as, but not limited to, boronic acids, boronate esters, or boronate salts), a base can be used (such as a strong base, for example, potassium tert-butoxide, potassium carbonate, or cesium carbonate) in a suitable solvent (such as polar aprotic solvents like dimethylformamide (DMF), toluene, or tetrahydrofuran (THF)). Further, such borylation reactions are typically carried out under inert atmosphere (argon or nitrogen) to prevent oxidation of the metal catalyst. Following the C-H borylation, bromination is carried out by reaction with a bromine agent, such as a bromide salt, such as, but not limited to, copper bromide, sodium bromide, potassium bromide, calcium bromide, or lithium bromide.

The oxidatively coupling step, used to form a dihydrophenanthrene core, can be carried out in the presence of a hypervalent iodine reagent and a Lewis acid catalyst. In some instances, the hypervalent iodine reagent is iodosobenzene bis(trifluoroacetate). Other hypervalent iodine reagents include, but are not limited to, iodobenzene diacetate, dess-Martin periodinane, trifluoroacetoxyiodobenzene, (diacetoxyiodo)benzene, iodosobenzene, iodine(III) trifluoroacetate, 2-iodoxybenzoic acid, or iodosylbenzene. In some instances, the Lewis acid catalyst is boron trifluoride etherate. Other suitable Lewis acid catalysts may be used.

In some instances, the demethylating of step (vi) is carried out in the presence of boron tribromide in a suitable solvent, such as dichloromethane. In some instances, other demethylation techniques may be used, such as but not limited to, demethylating conditions using strong acids (such as hydrochloric acid, sulfuric acid, or trifluoroacetic acid) at high temperature or reflux conditions in an appropriate solvent (such as polar aprotic solvents like dimethylformamide (DMF) or dimethyl sulfoxide (DMSO), or inert solvents like dichloromethane), which is typically performed under inert atmosphere (argon or nitrogen) to prevent oxidation or other side reactions.

Step (vii) of the method involves a Suzuki coupling reaction which replaces the bromo- group of 4-bromo-1,8-dimethyl-9,10-dihydrophenanthrene- 2,7-diol with a vinyl group to afford the final product (1,8-dimethyl-4-vinyl-9,10-dihydrophenanthrene-2,7-diol). In some instances, the vinyl trifluoroborate salt is potassium vinyltrifluoroborate and/or the palladium salt is Pd(dppf)Cl₂. Other suitable palladium salts can be used. In some instances, another type of coupling reaction can be used, in lieu of a Suzuki coupling These can include, without limitation, a Negishi coupling, a Heck reaction, a Stille coupling, or a Sonogashira coupling. The conditions for performing the aforementioned couplings are known to the skilled person.

The methods described include at least a purification of the final product. In some instances, the method includes the purification of the 1,2-bis(3-methoxy- 2-methylphenyl)ethane prior to step (iv); the 5-bromo-1-methoxy- 3-(3-methoxy-2-methylphenethyl)-2- methylbenzene prior to step (v); the 4-bromo-2,7-dimethoxy-1,8-dimethyl-9, 10- dihydrophenanthrene prior to step (vi); and/or the 4-bromo-1,8-dimethyl-9,10-dihydrophenanthrene-2,7-diol prior to step (vii). In some instances, the above purification(s) of intermediate or final product can be carried out by column chromatography. The intermediate and final product may also be purified by techniques, such as but not limited to, thin-layer chromatography, flash chromatography, high-performance liquid chromatography, preparative liquid chromatography, crystallization, recrystallization, liquid-liquid extraction, and distillation, as appropriate.

In some instances, the method produces the purified 1,8-dimethyl-4-vinyl-9,10-dihydrophenanthrene-2,7-diol of step (viii) in a total yield of at least about 1%, 5%, 10%, 15%, 20%, or 25% based on the amount of the 1-(bromomethyl)-3-methoxy-2-methylbenzene used in step (i). In some instances, the method produces the purified 1,8-dimethyl-4-vinyl-9,10-dihydrophenanthrene-2,7-diol of step (viii) in a total yield in a range from about 1% to 25% based on the amount of the 1-(bromomethyl)-3-methoxy-2-methylbenzene used in step (i).

For the methods described above, the synthetic conditions (choice of solvent(s), temperatures (such as to achieve refluxing), atmospheres, work-up conditions, purification conditions, etc.) can be varied or modified from those discussed and exemplified herein, as appropriate, by the person of ordinary skill in the art of synthetic chemistry, without impacting the synthesis. The methods may include additional steps, which are not otherwise specified, that may be involved in the syntheses which include, but are not limited to, precipitation of a product, washing the reaction mixture, extracting the reaction mixture, separation of the product from a mixture (e.g. by filtration), drying the product, and combinations thereof. Further processing steps that may be involved include stirring, heating and/or cooling the reaction mixture. Intermediate, crude, and purified products can be characterized using any suitable characterization method known to the skilled person. In some instances, such methods include, but are not limited to, ¹H and ¹³C NMR, mass spectrometry, elemental analysis, or other techniques.

The skilled person further understands that certain intermediate products of the method, as described above, may have one or more chiral centers and thus exist as one or more stereoisomers. As used herein, the term "stereoisomers" refers to compounds made up of the same atoms having the same bond order but having different three-dimensional arrangements of atoms which are not interchangeable. The term "chiral center" refers to a carbon atom to which four different groups are attached.

In some instances, the method further includes a step of forming a pharmaceutically acceptable salt of the purified 1,8-dimethyl-4-vinyl-9,10-dihydrophenanthrene-2,7-diol of step (viii), as described below.

### 1. Pharmaceutically Acceptable Salts

The 1-Methyleffusol compound may be converted into the form of a salt, such as a pharmaceutically acceptable salt. Examples of pharmaceutically acceptable salts include, but are not limited to, mineral or organic acid salts of basic residues such as amines; and alkali or organic salts of acidic residues such as carboxylic acids. The pharmaceutically acceptable salts include the conventional non-toxic salts or the quaternary ammonium salts of the parent compound formed, for example, from non-toxic inorganic or organic acids and inorganic or organic bases. Such conventional non-toxic salts include those derived from inorganic acids such as hydrochloric, hydrobromic, sulfuric, sulfamic, phosphoric, and nitric acids; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, tolunesulfonic, naphthalenesulfonic, methanesulfonic, ethane disulfonic, oxalic, and isethionic salts, and bases such as lithium hydroxide, sodium hydroxide, potassium hydroxide and ammonium hydroxide.

The pharmaceutically acceptable salts of the compounds can be synthesized from the 1-Methyleffusol compound by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are found in Remington's Pharmaceutical Sciences, 20th ed., Lippincott Williams & Wilkins, Baltimore, MD, 2000, p. 704; and "Handbook of Pharmaceutical Salts: Properties, Selection, and Use," P. Heinrich Stahl and Camille G. Wermuth, Eds., Wiley-VCH, Weinheim, 2002.

### A. Pharmaceutical Compositions

In some instances, the 1-Methyleffusol compound synthesized, as described herein, forms part of a pharmaceutical composition. The pharmaceutical composition includes an effective amount of the 1-Methyleffusol compound and optionally one or more pharmaceutically acceptable carriers and/or excipients.

In one non-limiting instance, a pharmaceutical composition includes:
1,8-dimethyl-4-vinyl-9,10-dihydrophenanthrene-2,7-diol or a pharmaceutically acceptable salt thereof; and
one or more pharmaceutically acceptable carriers; and
optionally one or more pharmaceutically acceptable excipients.

In some instances, the one or more pharmaceutically acceptable carriers are selected from water, ethanol, polyethylene glycol, propylene glycol, chitosan polymers and chitosan derivatives (e.g. N-trimethylene chloride chitosan, chitosan esters, chitosan modified with hydrophilic groups, such as amino groups, carboxyl groups, sulfate groups, etc.), methylcellulose, an oil, and combinations thereof. Other suitable carriers are known.

The pharmaceutical compositions include an effective amount of 1-Methyleffusol to promote bone formation by osteoblast cells. In some instances, the 1-Methyleffusol compound is present in a concentration ranging from about 1 µM to about 1 mM, from about 10 µM to about 500 µM, from about 10 µM to about 200 µM, or from about 25 µM to about 100 µM of the pharmaceutical composition, as well as individual values and sub-ranges contained within the aforementioned ranges. In some other instances, the 1-Methyleffusol compound is present in a concentration ranging from about 1 µM to about 10 mM, from about 1 µM to about 5 mM, from about 10 µM to about 1 mM, or from about 10 µM to about 500 µM of the pharmaceutical composition, as well as individual values and sub-ranges contained within the aforementioned ranges.

In some instances, depending on the route of administration, an effective amount of the 1-Methyleffusol compound in the composition can be between 0.01 to 1000 mg per kilogram body weight of the subject per day, between 0.1 and 500 mg, such as between 1 and 250 mg, for example about 5, 10, 20, 50, 100, 150, 200 or 250 mg, per kilogram body weight of the subject per day, which can be administered as a single daily dose, divided over one or more daily doses. The amount of the 1-Methyleffusol compound administered, the route of administration, and the further treatment regimen can be determined by the treating clinician or testing technologies, depending on factors such as the age, gender and general condition of the subject, the nature and severity of the disease/symptoms being prevented, treated, or diagnosed, and/or the samples being tested.

Pharmaceutical compositions containing a therapeutically effective amount of the 1-Methyleffusol compound described, and optionally one or more additional therapeutic, prophylactic, and/or diagnostic agents are provided. Pharmaceutical compositions can be for administration by parenteral (intramuscular, intraperitoneal, intravenous (IV), or subcutaneous injection), enteral, or transmucosal (intranasal, vaginal, rectal, or sublingual) routes of administration or using bioerodible inserts and can be formulated in dosage forms appropriate for each route of administration.

### i. Parenteral Compositions

Pharmaceutical compositions containing the 1-Methyleffusol compound described can be administered in an aqueous solution, by parenteral injection. Such compositions may also be in the form of a suspension or emulsion. In general, pharmaceutical compositions are provided including effective amounts of the active agent(s) (i.e., the 1-Methyleffusol compound and optionally include pharmaceutically acceptable diluents, preservatives, solubilizers, emulsifiers, adjuvants and/or carriers. Such compositions include diluents sterile water, buffered saline of various buffer content (e.g., Tris-HCl, acetate, phosphate), pH and ionic strength; and optionally, additives such as detergents and solubilizing agents (e.g., TWEEN^{®} 20, TWEEN^{®} 80 also referred to as polysorbate 20 or 80), antioxidants (e.g., ascorbic acid, sodium metabisulfite), and preservatives (e.g., Thimersol, benzyl alcohol) and bulking substances (e.g., lactose, mannitol). Examples of non-aqueous solvents or vehicles are propylene glycol, polyethylene glycol, vegetable oils, such as olive oil and corn oil, gelatin, and injectable organic esters such as ethyl oleate. The compositions may be lyophilized and redissolved/resuspended immediately before use. The compositions may be sterilized by, for example, filtration through a bacterium retaining filter, by incorporating sterilizing agents into the compositions, by irradiating the compositions, or by heating the compositions.

These particular aqueous solutions are especially suitable for intranasal or intratracheal administration to the primary infection site, such as the respiratory tract. These particular aqueous solutions are also suitable for intravenous, intramuscular, subcutaneous, and intraperitoneal administration. In this connection, sterile aqueous media which can be employed will be known to those of skill in the art in light of the present disclosure. For example, one dosage could be dissolved in 1 ml of isotonic NaCl solution and either added to variation in dosage will necessarily occur depending on the condition of the subject being treated. The person responsible for administration will, in any event, determine the appropriate dose for the individual subject.

### ii. Oral Compositions

The pharmaceutical composition containing the 1-Methyleffusol compound described may be provided in a form suitable for oral administration to a subject in need thereof, such as a mammal (i.e., an oral composition). Oral administration may involve swallowing, so that the 1-Methyleffusol compound and optionally additional active agent(s) enter the gastrointestinal tract, or buccal or sublingual administration may be employed by which the 1-Methyleffusol compound enter the blood stream directly from the mouth.

Compositions suitable for oral administration include solid compositions such as tablets, capsules containing particulates, liquids, powders, lozenges (including liquid-filled lozenges), chews, multi- and nano-particulates, gels, solid solutions, liposomes, films, ovules, sprays, and liquid compositions.

Liquid compositions for oral administration include suspensions, solutions, syrups, and elixirs. Such oral compositions may be employed as fillers in soft or hard capsules and can contain one or more suitable carriers and/or excipients, for example, water, ethanol, polyethylene glycol, propylene glycol, chitosan polymers and chitosan derivatives (e.g. N-trimethylene chloride chitosan, chitosan esters, chitosan modified with hydrophilic groups, such as amino groups, carboxyl groups, sulfate groups, etc.), methylcellulose, a suitable oil, one or more emulsifying agents, and/or suspending agents. Liquid compositions for oral administration may also be prepared by the reconstitution of a solid, for example, from a sachet.

For tablet or capsule dosage forms, in addition to the 1-Methyleffusol compound described herein, tablets generally contain disintegrants, binders, diluents, surface active agents, lubricants, glidants, antioxidants, colourants, flavoring agents, preservatives, or taste masking agents, or a combination thereof.

Examples of suitable disintegrants for forming a table or capsule dosage form containing the 1-Methyleffusol compound can include, but are not limited to, sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant can have a concentration in a range from about 1 wt% to about 25 wt%, from about 5 wt% to about 20 wt% of the tablet or capsule dosage form containing the 1-Methyleffusol compound.

Binders are generally used to impart cohesive qualities to a tablet composition containing the 1-Methyleffusol compound. Suitable binders for forming a tablet or capsule formulation can include, but are not limited to, microcrystalline cellulose, gelatin, sugars, polyethylene glycol, natural and synthetic gums, polyvinylpyrrolidone, pregelatinised starch, chitosan polymers and chitosan derivatives (e.g. N-trimethylene chloride chitosan, chitosan esters, chitosan modified with hydrophilic groups, such as amino groups, carboxyl groups, sulfate groups, etc.), hydroxypropyl cellulose, and hydroxypropyl methylcellulose.

Suitable diluents for forming a table or capsule composition containing the 1-Methyleffusol compound can include, but are not limited to, lactose (as, for example, the monohydrate, spray-dried monohydrate or anhydrous form), chitosan polymers and chitosan derivatives (e.g. N-trimethylene chloride chitosan, chitosan esters, chitosan modified with hydrophilic groups, such as amino groups, carboxyl groups, sulfate groups, etc.), N-sulfonated derivatives of chitosan, quaternarized derivatives of chitosan, carbosyalkylated chitosan, microcrystalline chitosan, mannitol, xylitol, dextrose, sucrose, sorbitol, microcrystalline cellulose, starch and dibasic calcium phosphate dihydrate.

Tablet or capsule compositions containing the 1-Methyleffusol compound may also contain surface active agents, such as sodium lauryl sulfate and polysorbate 80, and glidants such as silicon dioxide and talc, in the coating. When present, surface active agents can have a concentration in a range from about 0.2 wt% to 5 wt% of the tablet or capsule formulation.

Tablet or capsule compositions containing the 1-Methyleffusol compound also generally contain lubricants, such as magnesium stearate, calcium stearate, zinc stearate, sodium stearyl fumarate, and mixtures of magnesium stearate with sodium lauryl sulphate. Lubricants can have a concentration in a range from about 0.25 wt% to 10 wt%, from about 0.5 wt% to about 3 wt% of the tablet or capsule composition.

Other possible excipients included in a tablet or capsule formulation containing the 1-Methyleffusol compound include glidants (e.g. Talc or colloidal anhydrous silica at about 0.1 wt% to about 3 wt% of the table or capsule formulation), antioxidants, colourants, flavouring agents, preservatives, and taste-masking agents. When present, glidants can have a concentration in a range from about 0.2 wt% to 1 wt% of the tablet or capsule composition.

Tablet or capsule blends, including the 1-Methyleffusol compound and one or more suitable excipients, may be compressed directly or by roller to form tablets. Tablet or capsule blends or portions of the blends may alternatively be wet-, dry-, or melt-granulated, melt congealed, or extruded before tableting. The final table or capsule composition may contain one or more layers and may be coated or uncoated; it may even be encapsulated in a particle, such as a polymeric particle or a liposomal particle.

Solid formulations containing the 1-Methyleffusol compound for oral administration may be formulated to be immediate and/or modified release. Modified release formulations include delayed, sustained, pulsed, controlled, targeted and programmed release formulations.

### iii. Optional Therapeutic, Prophylactic or Diagnostic Agents

In some forms, the pharmaceutical compositions can optionally include one or more additional active agents. Therefore, in some forms, the pharmaceutical composition includes the 1-Methyleffusol compound, in addition to at least one of a therapeutic, prophylactic, and/or diagnostic agents. The additional agents can be included together with the 1-Methyleffusol compound or may be a separate composition for co-administration.

Non-limiting examples of therapeutic, prophylactic, or diagnostic agents can include bronchodilators, corticosteroids, methylxanthines, phosphodiesterase-4 inhibitors, anti-angiogenesis agents, antibiotics, antioxidants, anti-viral agents, anti-fungal agents, anti-inflammatory agents, immunosuppressant agents, anti-allergic agents, and combinations thereof. The amount of any additional therapeutic, prophylactic, or diagnostic agents generally depends on the severity of the diseases and/or disorders to be treated. Specific dosages can be readily determined by those of skill in the art. See Ansel, Howard C. et al., Pharmaceutical Dosage Forms and Drug Delivery Systems (6th ed.) Williams and Wilkins, Malvern, PA (1995).

### iv. Carriers and Excipients

The pharmaceutical compositions may be formulated with one or more excipients and/or carriers appropriate to the indicated route of administration. In some forms, the 1-Methyleffusol compound is formulated in a manner amenable for the treatment of human and/or veterinary subjects. In some forms, the pharmaceutical compositions include admixing or combining the 1-Methyleffusol compound with one or more of the following excipients: lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia, sodium alginate, polyvinylpyrrolidone, and/or polyvinyl alcohol. In some forms, e.g., for oral administration, the pharmaceutical compositions may be tableted or encapsulated. In some forms, the 1-Methyleffusol compound may be slurried in water, polyethylene glycol, propylene glycol, ethanol, corn oil, cottonseed oil, peanut oil, sesame oil, benzyl alcohol, sodium chloride, and/or various buffers. In some forms, the pharmaceutical compositions may be subjected to pharmaceutical operations, such as sterilization, and/or may contain carriers and/or excipients such as preservatives, stabilizers, wetting agents, emulsifiers, encapsulating agents such as lipids, dendrimers, polymers, proteins such as albumin, nucleic acids, and buffers.

### v. Dosage Forms

In some forms, it may be advantageous to formulate pharmaceutical compositions in dosage unit form for ease of administration and uniformity of dosage. "Dosage unit form" as used herein refers to physically discrete units suited as unitary dosages for the patients to be treated; each unit containing a predetermined quantity of therapeutic agent (i.e., the 1-Methyleffusol compound) calculated to produce the desired therapeutic effect in association with a pharmaceutical carrier. In some forms, the specification for the dosage unit forms dictated by and directly dependent on (a) the unique characteristics of the therapeutic agent and the particular therapeutic effect to be achieved, and (b) the limitations inherent in the art of compounding such a therapeutic agent for the treatment of a selected condition in a patient. In some forms, the 1-Methyleffusol compound are administered at a therapeutically effective dosage sufficient to treat a condition, such as an infection, associated with a condition in a patient. For example, the efficacy of a pharmaceutical composition containing the 1-Methyleffusol compound can be evaluated in an animal model system that may be predictive of efficacy in treating the disease in a human or another animal.

In some forms, the effective dose range for the therapeutic agent can be extrapolated from effective doses determined in animal studies for a variety of different animals. Precise amounts of the pharmaceutical composition depend on the judgment of the practitioner and are specific to each individual. Other factors affecting the dose include the physical and clinical state of the subject, the route of administration, the intended goal of treatment and the potency, stability, and toxicity of the particular pharmaceutical composition.

The actual dosage amount of an antibiotic composition of the present disclosure administered to a subject may be determined by physical and physiological factors such as type of animal treated, age, sex, body weight, severity of condition, the type of condition being treated, previous or concurrent therapeutic interventions, idiopathy of the patient and on the route of administration. These factors may be determined by a skilled artisan. The practitioner responsible for administration will typically determine the concentration of active agent(s) in a composition and appropriate dose(s) for the individual subject. The dosage may be adjusted by the individual physician in the event of any complication.

Single or multiple doses of the active agent(s), such as the 1-Methyleffusol compound, are contemplated. Desired time intervals for delivery of multiple doses can be determined by one of ordinary skill in the art employing no more than routine experimentation. As an example, patients may be administered two doses daily at approximately 12-hour intervals. In some forms, the agent is administered once a day.

The pharmaceutical composition may be administered on a routine schedule. As used herein, a routine schedule refers to a predetermined designated period of time. The routine schedule may encompass periods of time which are identical, or which differ in length, as long as the schedule is predetermined. For instance, the routine schedule may involve administration twice a day, every day, every two days, every three days, every four days, every five days, every six days, a weekly basis, a monthly basis or any set number of days or weeks there-between. Alternatively, the predetermined routine schedule may involve administration on a twice daily basis for the first week, followed by a daily basis for several months, etc. In other forms, the invention provides that the agent(s) may be taken orally and that the timing of which is or is not dependent upon food intake. Thus, for example, the agent can be taken every morning and/or every evening, regardless of when the patient has eaten or will eat.

In some forms, the pharmaceutical composition can be in a unit dosage form which can be suitably packaged, for example in a box, blister, vial, bottle, sachet, ampoule or in any other suitable single-dose or multi-dose holder or container (which can be properly labeled); optionally with one or more leaflets containing product information and/or instructions for use. Generally, such unit dosages can contain between 1 and 1000 mg, or between 5 and 500 mg, of the disclosed 1-Methyleffusol compound, e.g., about 10, 25, 50, 100, 200, 300 or 400 mg per unit dosage.

### III. Methods of Using 1-Methyleffusol and Pharmaceutical Compositions Thereof

The disclosed 1-Methyleffusol compound formed by the methods described can act as an osteoblast agent. The compound is typically administered in a pharmaceutical composition to a subject in need thereof. In one non-limiting example, method of stimulating bone formation comprising administering a pharmaceutical composition including an effective amount of 1-Methyleffusol to a subject in need thereof.

In some instances, the subject has osteoporosis. The method may also be used to promote bone formation in subjects which suffer from other diseases related with problems of the bone, such as weakening or weakened bones.

In some instances, the 1,8-dimethyl-4-vinyl-9,10-dihydrophenanthrene-2,7-diol, or pharmaceutically acceptable salt, used in the method can stimulate or induce alkaline phosphatase (ALP) activity in MC3T3-E1 osteoblasts, as compared to the method in the absence of the 1,8-dimethyl-4-vinyl-9,10-dihydrophenanthrene-2,7-diol or pharmaceutically acceptable salt.

In some instances, the 1,8-dimethyl-4-vinyl-9,10-dihydrophenanthrene-2,7-diol or pharmaceutically acceptable salt used in the method can promote calcium deposition and mineralization in MC3T3-E1 osteoblasts.

In some instances, the method can also reduce one or more symptoms of bone loss in the subject, as compared to the method in the absence of the 1,8-dimethyl-4-vinyl-9,10-dihydrophenanthrene-2,7-diol or pharmaceutically acceptable salt.

In some instances of the method, the pharmaceutical composition is administered to the subject by intranasal administration, oral administration, intramuscular administration, intravenous administration, intraperitoneal administration, subcutaneous administration, or a combination thereof.

In certain instances, in step (i) the dosage of the 1-Methyleffusol compound in the pharmaceutical composition administered to the subject is from about 0.1 µg to about 100 µg, from about 0.5 µg to about 50 µg, from about 1 µg to about 100 µg, from about 1 µg to about 50 µg, from about 1 µg to about 25 µg, from about 1 µg to about 10 µg, from about 1 µg to about 5 µg, from about 4 µg to about 10 µg, or from about 1 µg to about 4 µg per gram of the subject's weight, or sub-ranges or individual values contained within these ranges.

The administration in step (i) can be performed using any suitable technique, such as oral administration, intranasal, intramuscular administration, intravenous administration, intraperitoneal administration, or subcutaneous administration, or a combination thereof.

Step (i), administration of pharmaceutical composition, may occur one or more times. When more than one administration step is performed, each administration can be performed regularly every 5 mins, every 10 mins, every 20 mins, every 30 mins, every hour, every 2 hours, every day, every two days, every 3 days, every week, every two weeks, every month, etc.; or irregularly with a time interval of 5 mins, 10 mins, 20 mins, 30 mins, 1 hour, 2 hours, 1 day, 2 days, 3 days, 5 days, 1 week, 2 weeks, etc. The specific number of treatment and time interval for the treatment can be determined by the treating clinician or testing technologies, depending on factors such as the age, gender and general condition of the subject, the nature and severity of the disease/symptoms being prevented, treated, or diagnosed, and/or the samples being tested.

The administration step or all of the administration steps (if repeated), can each provide an effective amount of the 1-Methyleffusol compound in the pharmaceutical composition is administered to the subject, such that bacteria of the infection are killed and/or growth or proliferation of the bacteria are reduced or prevented, and thereby ameliorate one or more symptoms associated with the infection in the subject. Optionally, the treatment effect occurs without any notable side effects to the subject.

In some instances of the method, the pharmaceutical composition can further include one or more therapeutic, prophylactic, or diagnostic agents. In some other instances of the method, the method includes co-administering one or more therapeutic, prophylactic, or diagnostic agents to the subject, either before, during, or following step (i).

The present invention will be further understood by reference to the following non-limiting examples.

### EXAMPLES

### Example 1: Synthesis of 1,8-dimethyl-4-vinyl-9,10-dihydrophenanthrene-2,7-diol (also denoted 1-Methyleffusol)

### Synthetic Methods and Characterization:

### Synthesis of 1,2-bis(3-methoxy-2-methylphenyl)ethane (3):

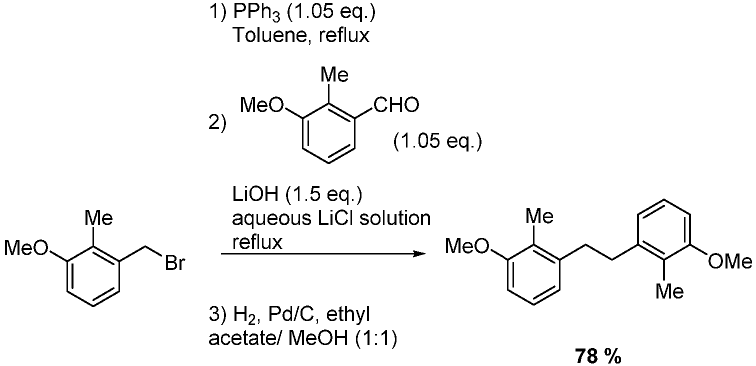

To a solution of triphenylphosphine (1.28 g, 4.88 mmol) in toluene (40 mL) was added 1-(bromomethyl)-3-methoxy-2-methylbenzene (1.00 g, 4.65 mmol). The mixture was heated to reflux overnight. The precipitate in reaction mixture was filtered and washed with toluene, and the crude product is air dried and used directly for next step. To a solution of crude phosphonium bromide in 1.4 M aqueous lithium chloride solution (20 mL) was added 3-methoxy-2-methylbenzaldehyde (0.72 g, 4.80 mmol) and lithium hydroxide (0.16 g, 6.85 mmol). The mixture was heated to reflux overnight. The reaction mixture was extracted with ethyl acetate thrice, the organic layers were combined, washed with brine solution, dried with sodium sulfate, filtered and concentrated, crude solid was used directly for next step. To a solution of crude alkene in 1: 1 ethyl acetate/methanol solution (14 mL) was added 10 % w/w palladium on charcoal (48.6 mg, 45.7 µmol), the mixture was stirred at room temperature under hydrogen atmosphere for overnight. The reaction mixture was filtered through celite, the filtrate was concentrated and purified by silica gel chromatography with 10 % ethyl acetate in hexane to afford 1,2-bis(3-methoxy-2-methylphenyl)ethane (0.98 g, 78 % yield). ¹H NMR (600 MHz, Chloroform-d) δ 7.12 (t, *J =* 7.9 Hz, 2H), 6.82 (d, *J =* 7.6 Hz, 2H), 6.74 (d, *J =* 8.1 Hz, 2H), 3.83 (s, 6H), 2.84 (s, 4H), 2.21 (s, 6H). ¹³C NMR (151 MHz, CDCl₃) δ 157.9, 141.8, 126.2, 124.7, 121.6, 108.2, 55.7, 34.9, 11.4. HRMS (ESI) Calcd. C₁₈H₂₃O₂ (M+H)⁺ m/z 271.1698, found 271.1693.

### Synthesis of 5-bromo-1-methoxy-3-(3-methoxy-2- methylphenethyl)-2-methylbenzene (4)

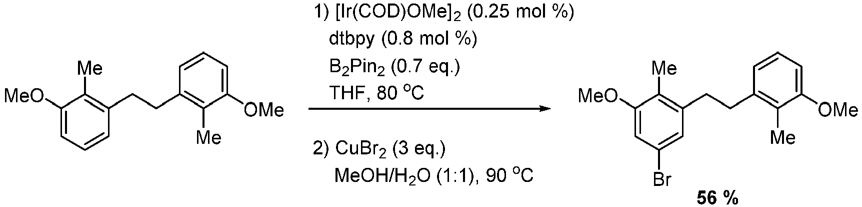

To a solution of 1,2-bis(3-methoxy-2-methylphenyl)ethane (8.25 g, 30.5 mmol) in THF (30 mL) was added bis(pinacolato)diboron (5.43 g, 21.4 mmol), 4,4'-Di-tert-butyl-2,2'- dipyridyl (65.5 mg, 0.244 mmol) and (1,5-cyclooctadiene)(methoxy)iridium(I) dimer (50.6 mg, 0.076 mmol). The mixture was purged with nitrogen and heated in a sealed tube at 80 °C overnight, the reaction mixture was evaporated and used directly for next step. To a solution of crude boronic ester in methanol (130 mL) was added copper(II) bromide (20.4 g, 91.5 mmol) in deionized water (130 mL), the mixture was heated at 80 °C for overnight. The reaction mixture was extracted with diethyl ether thrice, the organic layers were combined and washed with water and brine solution, dried over sodium sulfate and concentrated. The residue was purified by silica gel chromatography with 10 % ethyl acetate in hexane to afford 5-bromo-1-methoxy- 3-(3-methoxy-2-methylphenethyl)-2-methylbenzene (5.97 g, 56 % yield). ¹H NMR (600 MHz, Chloroform-d) δ 7.12 (t, *J* = 7.9 Hz, 1H), 6.96 (d, *J =* 1.9 Hz, 1H), 6.85 (d, *J =* 1.9 Hz, 1H), 6.79 (s, 1H), 6.75 (d, *J =* 8.2 Hz, 1H), 3.83 (s, 3H), 3.81 (s, 3H), 2.85 - 2.75 (m, 4H), 2.21 (s, 3H), 2.12 (s, 3H). ¹³C NMR (151 MHz, CDCl₃) δ 158.4, 157.8, 143.1, 141.1, 126.2, 124.5, 124.1, 123.7, 121.4, 119.1, 111.6, 108.2, 77.2, 77.0, 76.8, 55.8, 55.6, 34.5, 34.5, 11.3, 11.1. HRMS (ESI) Calcd. C₁₈H₂₂BrO₂ (M+H)⁺ m/z 349.0803, found 349.0794.

### Synthesis of 4-bromo-2,7-dimethoxy-1,8-dimethyl-9,10-dihydrophenanthrene (5)

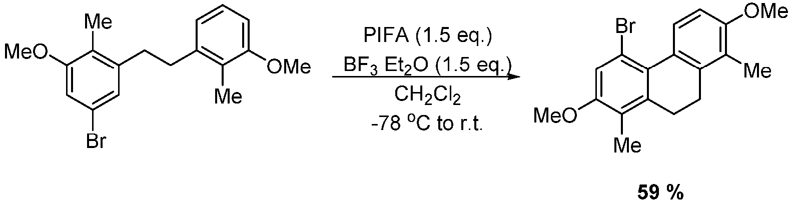

To a solution of 5-bromo-1-methoxy-3-(3-methoxy-2-methylphenethyl)-2-methylbenzene (1.00 g, 2.86 mmol) in CH₂Cl₂ (40 mL) was added dropwise the CH₂Cl₂ (10 mL) solution of iodosobenzene bis(trifluoroacetate) (1.84 g, 4.29 mmol) and boron trifluoride etherate (0.53 mL, 4.29 mmol) at -78 °C. The reaction mixture was stirred at - 40 °C overnight, evaporated and purified by column chromatography with 10 % ethyl acetate in hexane to afford 4-bromo-2,7-dimethoxy-1,8-dimethyl-9,10-dihydrophenanthrene (0.59 g, 59 % yield). ¹H NMR (600 MHz, Chloroform-d) δ 8.01 (d, *J* = 8.7 Hz, 1H), 7.04 (s, 1H), 6.80 (d, *J =* 8.7 Hz, 1H), 3.87 (s, 3H), 3.84 (s, 3H), 2.75 - 2.64 (m, 4H), 2.23 (s, 3H), 2.17 (s, 3H). ¹³C NMR (151 MHz, CDCl₃) δ 156.7, 156.2, 140.8, 139.1, 128.1, 127.0, 126.6, 122.7, 122.3, 117.5, 114.8, 106.8, 55.9, 55.6, 27.1, 25.6, 11.8, 11.7. HRMS (ESI) Calcd. C₁₈H₂₀BrO₂ (M+H)⁺ m/z 347.0647, found 347.0640.

### Synthesis of 4-bromo-1,8-dimethyl-9,10-dihydrophenanthrene- 2,7-diol

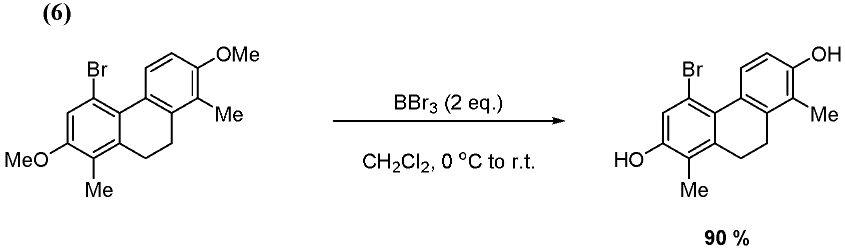

To a solution of 4-bromo-2,7-dimethoxy-1,8-dimethyl-9,10-dihydrophenanthrene (0.67 g, 1.92 mmol) in CH₂Cl₂ (20 mL) was added dropwise 1 M boron tribromide in CH₂Cl₂ (3.85 mL, 3.85 mmol) in ice bath. The reaction mixture was added methanol (10 mL) in ice bath and stirred for 15 min, evaporated under reduced pressure and purified by column chromatography with 30 % ethyl acetate in hexane to afford 4-bromo-1,8-dimethyl-9,10-dihydrophenanthrene-2,7-diol (0.55 g, 90 % yield). ¹H NMR (600 MHz, Chloroform-d) δ 7.91 (d, *J =* 8.5 Hz, 1H), 7.03 (s, 1H), 6.71 (d, *J =* 8.5 Hz, 1H), 4.78 (s, 1H), 4.75 (s, 1H), 2.69 (brs, 4H), 2.26 (s, 3H), 2.20 (s, 3H). ¹³C NMR (151 MHz, CDCl₃) δ 152.6, 152.2, 141.1, 139.3, 128.3, 127.0, 126.8, 120.5, 119.9, 119.0, 117.0, 111.5, 27.0, 25.4, 11.7, 11.6. HRMS (ESI) Calcd. C₁₆H₁₆BrO₂ (M+H)⁺ m/z 319.0334, found 319.0327.

### Synthesis of 1,8-dimethyl-4-vinyl-9,10-dihydrophenanthrene-2,7-diol (1-methyleffusol)

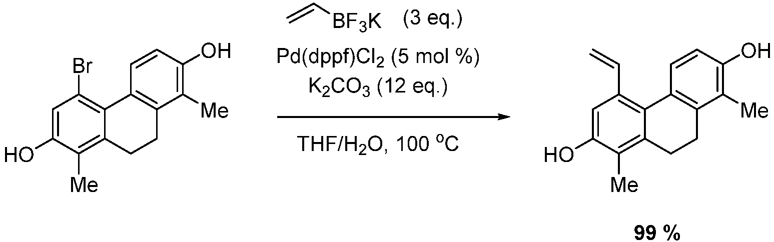

To a solution of 4-bromo-1,8-dimethyl-9,10-dihydrophenanthrene-2,7-diol (0.20 g, 0.627 mmol) in 1.4:1 THF/deionized water (10.5 mL) was added potassium carbonate (1.04 g, 7.52 mmol), potassium vinyltrifluoroborate (0.28 g, 2.07 mmol) and Pd(dppf)Cl₂ (22.9 mg, 0.031 mmol). The mixture was purged with nitrogen and heated in sealed tube at 100 °C overnight. The reaction mixture was acidified by 1 M HCl solution and extracted with CH₂Cl₂ thrice, the combined organic layers were washed with brine solution and dried over sodium sulfate, purified by column chromatography with 20 % ethyl acetate in hexane to afford 1,8-dimethyl-4-vinyl- 9,10-dihydrophenanthrene-2,7-diol (0.165 g, 99 % yield). ¹H NMR (600 MHz, Chloroform-d) δ 7.26 (d, *J =* 8.3 Hz, 1H), 6.91 (dd, *J =* 17.4, 10.7 Hz, 1H), 6.87 (s, 1H), 6.67 (d, *J =* 8.3 Hz, 1H), 5.60 (d, *J=* 17.2 Hz, 1H), 5.18 (d, *J =* 10.7 Hz, 1H), 5.08 (s, 1H), 5.06 (s, 1H), 2.76 - 2.62 (m, 4H), 2.27 (s, 3H), 2.24 (s, 3H). ¹³C NMR (151 MHz, CDCl₃) δ 152.3, 152.1, 139.3, 139.1, 138.6, 133.6, 127.9, 127.7, 127.4, 120.7, 120.3, 113.2, 112.0, 111.8, 26.2, 25.4, 11.7, 11.6. HRMS (ESI) Calcd. C₁₈H₁₈O₂ (M⁺) m/z 266.1307, found 266.1301.

### Results and Discussion:

The synthesis of 1-methyleffusol involved sequential synthetic steps shown in Scheme A below. Briefly, commercially available benzyl bromide **1** was initially reacted with triphenylphosphine to yield dianisole **3**, followed by a Wittig reaction with the corresponding aldehyde **2** and subsequent hydrogenation. Notably, these conversions did not require chromatographic purification and resulted in a 78% yield of compound **3.**

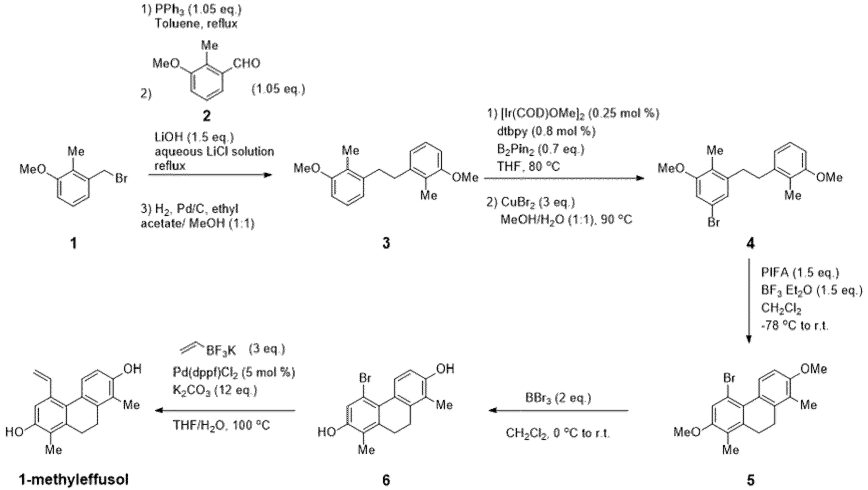

### Scheme A. Synthetic route of 1,8-dimethyl-4-vinyl-9,10-dihydrophenanthrene-2,7-diol

Dianisole **3** underwent C-H borylation using an iridium(I) catalyst and subsequent bromination, resulting in a 56% yield of bromide **4**. The phenanthrene core of compound **5** was formed through oxidative coupling of bromide **4** under hypervalent iodine reagent and boron trifluoride etherate condition.

To complete the synthesis, demethylation utilizing boron tribromide and a Suzuki coupling with vinyl trifluoroborate salts were carried out, resulting in the desired natural product, 1-methyleffusol. Both the proton and carbon NMR spectra of the synthesized compound matched the literature.

Overall, this synthetic route achieved a total yield of 23% starting from the corresponding benzyl bromide, providing for an efficient production of 1-methyleffusol. The synthetic approach developed included five overall synthetic steps making a large scale production of 1-methyleffusol possible, when compared to the traditionally used isolation approach, which is tedious and labor-intensive providing 1-Methyleffusol only in tiny quantities.

### Example 2: Stimulation of Bone Forming Activity by 1-Methyleffusol

1-Methyleffusol was synthesized according to the method of Example 1 above. In prior studies, it was found that 1-methyleffusol exhibited the ability for stimulation of bone forming activities (U.S. Application No. 17/713,384; and Chinese Application No. 202210551690.4).

### Stimulation of ALP Activity:

Cells were treated with 0.5 µM and 1.5 µM concentrations of 1-Methyleffusol for 4 days and the alkaline phosphatase (ALP) activity, as a marker of bone forming activity, was measured. Ascorbate and beta-glycerophosphate treatment (ASC+b-GP) was performed as positive control for ALP induction. As shown in the Figure, 1-Methyleffusol was found to stimulate ALP activity.

### Promotion of Osteoblast Mineralization:

Cells were treated with 1-Methyleffusol (1.5 or 5 mM) in osteoblast mineralization medium (OM) for 14 days, and the calcium deposition in the cell layer was measured by Alizarin Red staining (images not shown). It was found that 1-Methyleffusol promoted mineralization in MC3T3-E1 osteoblasts.

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific instances of the invention described herein. Such equivalents are intended to be encompassed by the following claims.

## Claims

1. A method of making 1,8-dimethyl-4-vinyl-9,10-dihydrophenanthrene-2,7-diol, the method comprising the steps of:
(i) refluxing 1-(bromomethyl)-3-methoxy-2-methylbenzene and triphenylphosphine in solution to afford a phosphonium bromide;
(ii) refluxing the phosphonium bromide and 3-methoxy-2-methylbenzaldehyde in an alkaline solution to afford (Z)- and (E)-1,2-bis(3-methoxy-2-methylphenyl)ethene stereoisomers;
(iii) hydrogenating the (Z)- and (E)-1,2-bis(3-methoxy-2-methylphenyl)ethene to afford 1,2-bis(3-methoxy- 2-methylphenyl)ethane stereoisomers;
(iv) brominating the 1,2-bis(3-methoxy- 2-methylphenyl)ethane to afford 5-bromo-1-methoxy-3-(3-methoxy-2-methylphenethyl)-2-methylbenzene;
(v) oxidatively coupling the 5-bromo-1-methoxy-3-(3-methoxy-2- methylphenethyl)-2-methylbenzene to afford 4-bromo-2,7-dimethoxy-1,8-dimethyl-9,10- dihydrophenanthrene;
(vi) demethylating the 4-bromo-2,7-dimethoxy-1,8-dimethyl-9,10-dihydrophenanthrene to afford 4-bromo-1,8-dimethyl-9,10-dihydrophenanthrene- 2,7-diol;
(vii) subjecting the 4-bromo-1,8-dimethyl-9,10-dihydrophenanthrene- 2,7-diol to a Suzuki-coupling reaction with a vinyl trifluoroborate salt and a palladium salt to afford 1,8-dimethyl-4-vinyl-9,10-dihydrophenanthrene-2,7-diol; and
(viii) purifying the 1,8-dimethyl-4-vinyl-9,10-dihydrophenanthrene-2,7-diol.

2. The method of claim 1, wherein the refluxing of step (i) occurs in toluene.

3. The method of claim 1, wherein the alkaline solution is an aqueous solution comprising lithium chloride and lithium hydroxide.

4. The method of claim 1, wherein the hydrogenating of step (iii) comprises reacting the (E)-1,2-bis(3-methoxy-2-methylphenyl)ethene with palladium on charcoal in one or more solvents under a hydrogen gas atmosphere.

5. The method of claim 1, wherein the brominating of step (iv) occurs by C-H borylation using an iridium(I) catalyst and a boron agent, and subsequent bromination with a bromide salt.

6. The method of claim 1, wherein the oxidatively coupling of step (v) is carried out in the presence of a hypervalent iodine reagent and a Lewis acid catalyst.

7. The method of claim 1, wherein the demethylating of step (vi) is carried out in the presence of boron tribromide.

8. The method of claim 1, wherein the vinyl trifluoroborate salt is potassium vinyltrifluoroborate and/or the palladium salt is Pd(dppf)Cl₂.

9. The method of claim 1, wherein:
- the 1,2-bis(3-methoxy- 2-methylphenyl)ethane is subjected to purification prior to step (iv);
- the 5-bromo-1-methoxy- 3-(3-methoxy-2-methylphenethyl)-2- methylbenzene is subjected to purification prior to step (v);
- the 4-bromo-2,7-dimethoxy-1,8-dimethyl-9,10- dihydrophenanthrene is subjected to purification prior to step (vi); and/or
- the 4-bromo-1,8-dimethyl-9,10-dihydrophenanthrene-2,7-diol is subjected to purification prior to step (vii).

10. The method of claim 1, further comprising a step of forming a pharmaceutically acceptable salt of the purified 1,8-dimethyl-4-vinyl-9,10-dihydrophenanthrene-2,7-diol of step (viii).

11. A pharmaceutical composition comprising:
1,8-dimethyl-4-vinyl-9,10-dihydrophenanthrene-2,7-diol or a pharmaceutically acceptable salt thereof; and
one or more pharmaceutically acceptable carriers; and
optionally one or more pharmaceutically acceptable excipients.

12. The pharmaceutical composition of claim 11, wherein the one or more pharmaceutically acceptable carriers are selected from the group consisting of water, ethanol, polyethylene glycol, propylene glycol, chitosan polymers and chitosan derivatives, methylcellulose, and an oil.

13. The pharmaceutical composition of claim 15, wherein the 1,8-dimethyl-4-vinyl-9,10-dihydrophenanthrene-2,7-diol or pharmaceutically acceptable salt thereof is at a concentration ranging from about 1 µM to about 10 mM, from about 1 µM to about 5 mM, from about 10 µM to about 1 mM, or from about 10 µM to about 500 µM of the pharmaceutical composition.

14. A method of stimulating bone formation comprising administering the pharmaceutical composition of claim 15 to a subject in need thereof.

15. The method of claim 14, wherein the subject has osteoporosis.

16. The method of claim 14, wherein the pharmaceutical composition is administered to the subject by intranasal administration, oral administration, intramuscular administration, intravenous administration, intraperitoneal administration, subcutaneous administration, or a combination thereof.

17. The method of claim 14, wherein the 1,8-dimethyl-4-vinyl-9,10-dihydrophenanthrene-2,7-diol, or pharmaceutically acceptable salt, used in the method stimulates or induces alkaline phosphatase (ALP) activity in MC3T3-E1 osteoblasts, as compared to the method in the absence of the 1,8-dimethyl-4-vinyl-9,10-dihydrophenanthrene-2,7-diol or pharmaceutically acceptable salt.

18. The method of claim 14, wherein the 1,8-dimethyl-4-vinyl-9,10-dihydrophenanthrene-2,7-diol or pharmaceutically acceptable salt used in the method promotes calcium deposition and mineralization in MC3T3-E1 osteoblasts.

19. The method of claim 18, wherein the method also reduces one or more symptoms of bone loss in the subject, as compared to the method in the absence of the 1,8-dimethyl-4-vinyl-9,10-dihydrophenanthrene-2,7-diol or pharmaceutically acceptable salt.

20. A compound having a chemical structure of Formula (I): 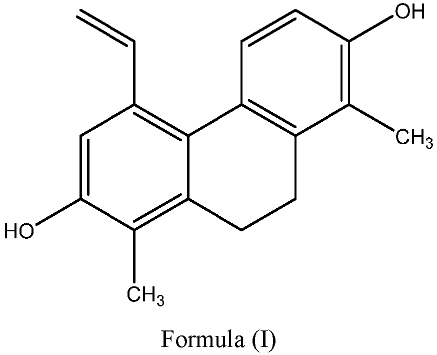 wherein the compound is synthesized according to the method of claim 1.
